Europäisches Patentamt

European Patent Office

Office européen des brevets

(19)

(11) Publication number: **0 275 653 B1**

# EUROPEAN PATENT SPECIFICATION

(12)

(45) Date of publication of patent specification: **03.07.91**  (51) Int. Cl.5: **A61F 2/06, A61L 27/00**

(21) Application number: **87310854.2**

(22) Date of filing: **10.12.87**

(54) Vascular prosthesis.

(30) Priority: **07.01.87 GB 8700249**

(43) Date of publication of application:
**27.07.88 Bulletin 88/30**

(45) Publication of the grant of the patent:
**03.07.91 Bulletin 91/27**

(84) Designated Contracting States:
**AT BE CH DE ES FR GB IT LI LU NL SE**

(56) References cited:
**FR-A- 2 522 696**
**GB-A- 1 552 388**
**US-A- 4 208 745**

**TRANSACTIONS OF THE AMERICAN SOCI-
ETY FOR ARTIFICIAL INTERNAL ORGANS,
vol. 29, 28th - 30th April 1983, Toronto, CA,
pages 255-259, Lovettsville, Virginia, US; E.
LOMMEN et al.: "Development of a neo-
artery induced by a biodegradable polymer-
ic vascular prosthesis"**

(73) Proprietor: **IMPERIAL CHEMICAL INDUSTRIES
PLC
Imperial Chemical House, Millbank
London SW1P 3JF(GB)**

(72) Inventor: **Hayman, Nigel Ward
3 Bleasby Gardens Lansdown Road
Cheltenham Gloucestershire GL52 6UL(GB)**

(74) Representative: **Hulse, Raymond et al
Imperial Chemical Industries PLC Legal De-
partment: Patents PO Box 6 Bessemer Road
Welwyn Garden City Hertfordshire AL7
1HD(GB)**

## Description

This invention relates to the field of porous vascular graft prostheses and more particularly to a melt spun hollow fibrous vascular prosthesis.

There are three reasons for replacing a human artery or vein; disease, deterioration with age or trauma, ie accidental damage. Of these the first two are the principal causes as confirmed by the average age of recipients being 67 years.

Prior to the use of a synthetic vascular prosthesis, there were three options open to the surgeon. Post mortem removal from human or animal sources was possible but, without extensive pretreatment, this usually led to rejection by the immune system. The third option, replacement by the patients own saphenous vein, is the preferred route. However, this is not always feasible since it may also be diseased, removed previously or, in cases of trauma, there may be insufficient time. Thus there is a continuing need for a synthetic vascular prosthesis.

At present prostheses of woven or knitted polyester fabrics and porous polytetrafluoroethylene tubes (see Australian Patent No 549,620) are available. Though these may be used for smaller diameter grafts, their success rate diminishes with diameter. The reason for this lies in the 'foreign body' response they provoke from the host. The body's response to an implant is to recognise it as a foreign object which must be destroyed. Through a complicated series of steps, this leads to formation of thrombis, ie clots in the artery. With the larger diameter prostheses, blood flow is sufficiently high and non turbulent to minimise this problem. As the diameter decreases, both blood flow and turbulence increase, dislodging material. Thus the performance of the prosthesis drops sharply with diameter.

French Patent 2,522,692 on which the preamble of Claim 1 is based, describes a porous tubular product suitable for use as a vascular graft. The tube is produced by simultaneously extruding from a spinneret a polymer solution into a number (six) of filament streams and on to a rotating mandrel. The spinneret is reciprocally driven relative to the mandrel to provide an angle of winding of 45°. A sufficient number of passes is made to produce the desired wall thickness with overlapping filaments bonding to each other. Solvent is removed from the tube by first heating it and then by immersion in water.

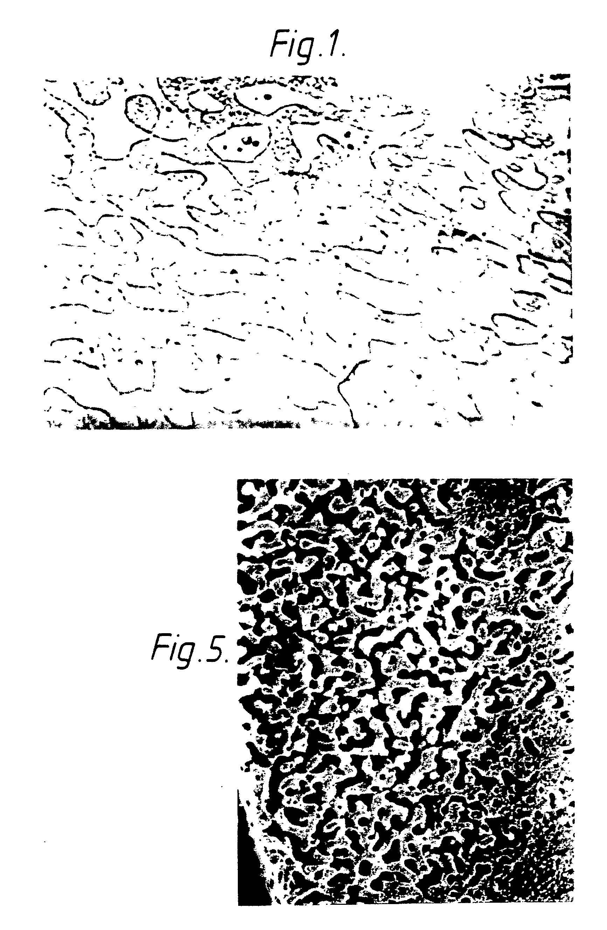

It is apparent that the filaments in the tube are (1) not aligned to the axis of tube (see in particular Figs 1 and 10 which clearly show that the filaments are strongly aligned in two directions, both directions being offset by a constant angle to the axis of the tube such angle being dependent on the speed of rotating mandrel and the speed of the reciprocating spinneret), (2) not attached to each other in a random manner.

United States Patent 4,208,745 is concerned with a tubular vascular prosthesis made from polytetrafluoroethylene. The tubular prosthesis is formed by (1) extruding a mixture of polytetrafluoroethylene and white oil to form a tube, (2) heating the tube to remove the white oil, (3) stretching the tube in a longitudinal direction to produce fibres and nodes therein, and (4) sintering the tube. The fibres which are formed in the tube are aligned orthogonally to the direction of stretch ie orthogonally to the axis of the tube.

The histology of an implant can be summarised as follows: the principle requirement is biocompatibility ie, the implant must not provoke a 'foreign body' reaction beyond that normally associated with a surgical wound. As part of that normal healing reaction, antibodies will swarm around the wound and implant site to engulf the foreign body. These will be followed by fibroblast cells which will extude collagen to form the skeleton upon which new, living tissue will form. In this process the collagenous scar tissue becomes vascularised, ie suffused by tiny blood vessels which enrich this new lining. Upon this wall will form a thin layer of cells, the endothelium, a naturally non-thrombogenic surface. If, during formation, this new tissue fails to anchor properly then material can break off and block the artery downstream. However, this tissue ingrowth needs to be limited otherwise scar tissue will grow through the prosthesis wall so rendering it stiff and non-compliant. Also this fibrotic response must cease at a finite time, otherwise it will expand into the prosthesis interior as a potential occlusion. The degree of tissue ingrowth can be controlled by wall porosity and choice of graft material.

Parallel to these rigorous biochemical requirements are an equally demanding call upon the physical properties of the prosthesis. The close matching of mechanical properties between vascular graft and natural vessel is essential for long term potency. The pressure wave from the heart must travel smoothly through the anastomosis with minimum reflection. If a smooth transition is not achieved high shear stresses are placed on the suture line, increasing the possibility of failure. In addition the resulting turbulence is believed to produce increased platelet action and endothelial cell damage, leading to possible thrombus.

The matching of mechnical properties is not simple. Natural vessels are complex, non-linear, will be of varying age and type, and are likely to be diseased with substantially different physical properties to those of healthy vessels. In addition fibrous tissue ingrowth into the porous surface of the graft after implantation may substantially alter its physical properties so adding to this highly complex problem.

It is unclear, at present, how a vascular graft must be characterised before implantation. However, there are some general guidelines. It must be compliant, exhibiting a high degree of flexibility both longitudinally and radially. It must withstand internal pressures of typically 150 mm Hg. It must be pulsatile, capable of transmitting the pulse of a heart beat at around 45 million cycles per year. A natural artery may be asked to withstand, elastically, strain of up to 50%. But it must not do this with 'rubber' elasticity as this may lead to aneurism, the catastrophic, balloon-like, deformation.

A synthetic vascular prosthesis requires to be made from a material which is biocompatible and preferably has a proven history of implant use; optimally in blood contact applications. It must be compliant and pulsatile, but not be prone to aneurism. It should be viscoelastic and exhibit good flex fatigue resistance. The prosthesis should also be microporous to an extent which provides a firm anchorage for the collagen but on the other hand prevents excessive tissue ingrowth which would lead to non compliance and/or occlusion. The prosthesis should also be conformable without 'kinking' at any point. A small pore size of less than 50 $\mu$m may be beneficial in obtaining sufficient tissue incorporation whilst still maintaining high compliance.

The necessary degree of porosity and also pore size distribution for optimum healing and long-term potency is still to be determined. Indeed a non symmetrical pore distribution may be beneficial.

According to the present invention we provide a porous tubular, substantially cylindrical synthetic vascular prosthesis, the wall of which has a structure comprising spaced fibrils of a synthetic polymeric material, characterised in that the tube has been produced by melt spinning the polymeric material and the spaced fibrils of synthetic polymeric material are substantially aligned to the axis of the tube, such aligned spaced fibrils being interconnected to each other in a random manner.

It is believed that in the invention because the pores in the wall of the prosthesis are highly oriented in the direction of the tube ie in the direction of spinning this will form a more tortuous, porous network than associated with a woven or knitted fabric. It is believed that this will substantially inhibit tissue growth through the prosthesis wall.

Also, because of the nature of the melt spinning process used to produce the prosthesis of the invention we believe that the size and uniformity of the pores in the wall of the prosthesis can be controlled within close limits so that when grafted the prosthesis has a high efficiency.

The prosthesis of the invention can be of any suitable fibre-forming melt spinnable polymer which is chemically inert and body and blood compatible. A particularly desirable polymer is a polyether based polyurethane and more particularly one which is based on poly-(tetrahydrofuran), methylene bis (4-phenylisocyanate) and 1,4-butane diol and the suitability of such polyurethanes as materials for vascular prosthesis are supported by D Annis et al in Vol XXIV Trans.Am.Soc.Artif Intern organs. 1978 209. Other polyurethanes which may be used are those based on other glycol residues such as polyethylene glycol which will introduce a degree of hydrophilicity to the prosthesis.

Apart from the prosthesis being of a polyurethane, we envisage that it could also be of such fibre forming polymers as polypropylene, polyethylene, polyester, nylon fluoropolymers, polystyrene, polyvinylchloride, polymethylmethacrylate, cellulose acetate butyrate.

Also, by careful selection of polymer, the prosthesis may be rendered biodegradable which may offer a considerable advantage. Examples of suitable polymers are polyethylene oxide, polyethylene terephthalate, polydioxanone, polyglycolic acid.

The prosthesis of the invention can be made in any size including diameter, wall thickness and length as required for cardio-vascular surgery.

Though the prosthesis of the invention may be used in isolation it may be found more desirable to use the prosthesis in combination with an external cladding of a woven or knitted fabric of the type currently used in the prior art.

Furthermore it may be desirable to treat the prosthesis with suitable anti-coagulants before the graft is carried out. In addition it may also be desirable at an appropriate stage of manufacture and at implantation to ensure that the prosthesis has been sterilised as for example by the use of gamma radiation (eg cobalt - 60 source) or ethylene oxide gas.

The invention will now be described with the aid of examples.

In the following examples prosthetic tubes were fabricated using a small screw extruder fitted with a standard pack assembly. Various spinneret designs may be used in the fabrication process.

## EXAMPLE 1

In this example the blend composition was 43% by weight of polypropylene (melt viscosity 580 poise at 284°C), 53% by weight of nylon 66 (melt viscosity 800 poise at 284°C) and 4% of nylon 11 (Rilsan - melt viscosity 500 poise at 284°C) as compatabiliser. Before spinning the nylon 66 and nylon 11 were both dried for 16 hours under vacuum at 80°C.

The blend was spun at a temperature of 282° with a throughput of 4.4 grams/minute, through a double C spinneret. The spinneret orifice had a width of 100 μm and a spacing of 250 μm. The tube was wound up at 17.5 metres/minute. The tube was quenched using a conventional air quench.

Samples of the tube were immersed, with agitation, in 98% formic acid for 2½ hours. Substantially all of the nylon (66 and 11) was removed from the wall of the tube leaving spaced fibrils of polypropylene which are substantially aligned to the axis of the tube, such fibrils being interconnected to each other in a random manner. Fig 1 shows a photomicrograph cross-section of the unextracted tube.

## EXAMPLE 2

In this example a blend was formed between 65% by weight of a polyether based polyurethane (Estane 58300 - B F Goodrich) and 35% by weight of polypropylene (Propathene PXC 31631). The polyurethane had been dried under vacuum for 2½ hours at 96° C. Melt viscosities for the polyether based polyurethane and polypropylene at 190° C were 10340 poise and 1980 poise respectively. The blend was spun at a temperature of 190° C through a double C spinneret, the legs of the individual C's being in juxtaposition, with a throughput of 8.7 grams per minute to produce a hollow tube having an outside diameter of 3.9 mm and an inside diameter of 2.8 mm. The dimensions of the 2C spinneret were: internal radius 1.09 mm, width of slot 0.16 mm and a gap of 0.38 mm.

The tube was quenched using a conventional air quench.

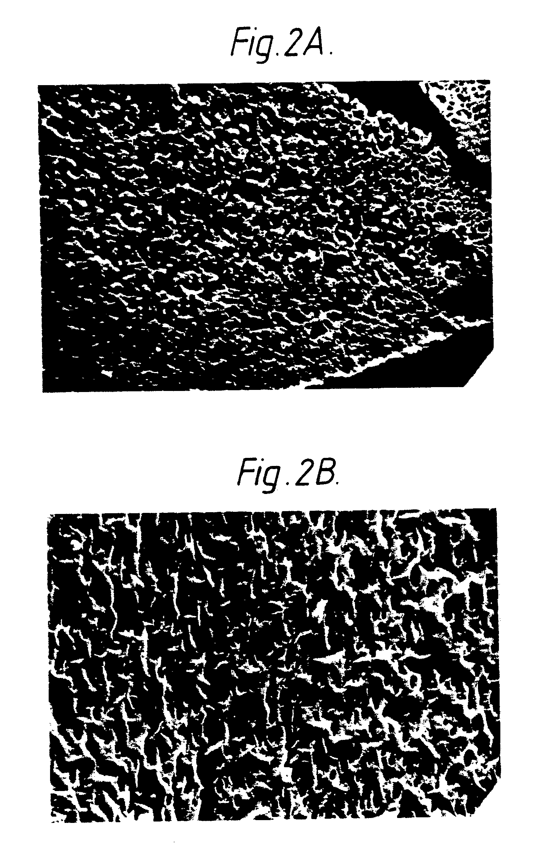

Samples of the tube were immersed in 100 cc of boiling petroleum in a flask for 45 minutes. The solution was decanted off and replenished with 100 ccs of petroleum ether and boiled for a further 60 minutes. Substantially all of the polypropylene was extracted from the wall of the tube leaving a wall having an interior structure comprising spaced fibrils of polyurethane which were substantially aligned to the acis of the tube; such aligned spaced fibrils being interconnected to each other in a random manner. The interior structure of the wall is shown in the accompanying photomicrographs. Fig 2a shows a cross-section view of the wall. Fig 2b shows a longitudinal view of the substantially aligned interconnected fibrils.

The tube was white, opaque, integral, elastic and was ideally suited as a vascular prosthesis.

## EXAMPLE 3

A blend was formed between 65% by weight of a polyester based polyurethane (Estane 58271 ex B F Goodrich) and 35% by weight of polypropylene (Propathene PXC 316313). The polyurethane was dried for 3 hours at 70° C under vacuum. Melt viscosities for the polyurethane and the polypropylene at 180° C were 43850 poise and 2790 poise respectively.

The blend was spun at a temperature of 180° C through a double C spinneret, the legs of the individual C's being in juxtaposition, with a throughput of 3.2 grams per minute to produce a hollow tube having an outside diameter of 3 mm and an inside diameter of 2 mm. The two C spinnerets had dimensions of 1.09 mm internal radius, width of slot 0.16 mm and a gap of 0.38 mm.

The tube was quenched using a conventional air quench.

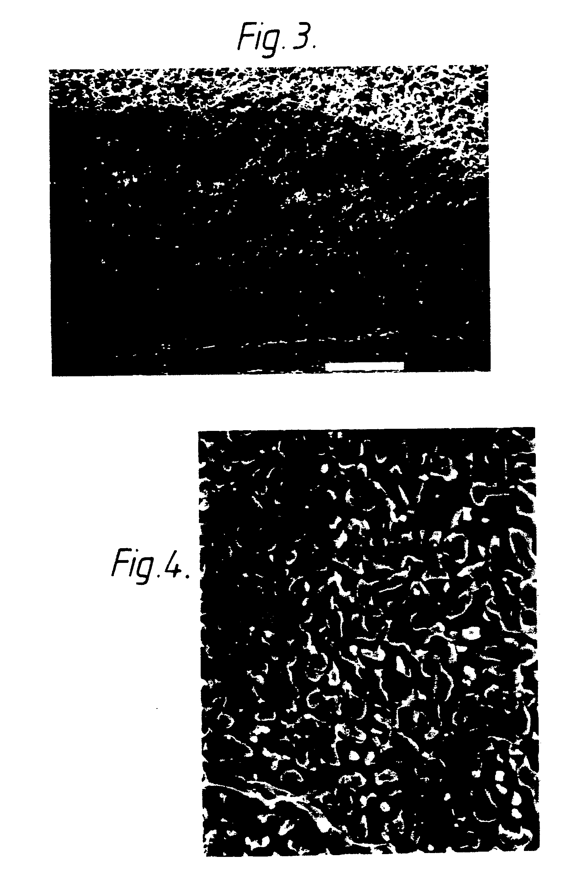

500 mg samples of the tube were immersed in 80 cc of boiling petroleum in a flask for 105 minutes. The solution was decanted off and replenished with 100 ccs of petroleum ether and boiled for a further 60 minutes. Substantially all of the polypropylene was extracted from the wall of the tube leaving a wall having an interior structure comprising spaced fibrils of polyurethane which were substantially aligned to the axis of the tube such aligned spaced fibrils being intereconnected to each other in a random manner. The interior structure of the wall is shown in the accompanying photomicrograph. Fig 3 shows a cross-sectional view of the wall.

The tube was white, opaque, integral, elastic and was ideally suited as a vascular prosthesis.

## EXAMPLE 4

In this example a blend was formed between 65% by weight of polyether based polyurethane (Estane 58300) and 35% by weight of polypropylene (Propathene PXC 31631). The polyurethane was dried under vacuum at 62° C for 4 hours. Melt viscosities for the polyurethane and the polypropylene at 190° C were 15620 poise and 2017 poise respectively. The blend was spun at 190° C through a double C spinneret. The C spinneret had an inner radius of 1.09 mm, a slot width of 0.16 mm and a gap of 0.38 mm.

The throughput was 8.7 g/min. The tube was quenched using a conventional air quench.

300 mg samples were placed in 50 cc of cyclohexanone (GPR grade) and shaken for 4½ hours at room temperature. Substantially all of the polyurethane was extracted from the wall of the tube leaving a wall having an interior structure comprising of spaced fibrils of polypropylene which were substantially aligned to the axis of the tube. These fibrils were interconnected to each other in a random manner. The interior structure of the wall is shown in the accompanying photomicrograph Fig 4.

The tube was white, opaque, integral, elastic and suitable for use as a vascular prosthesis.

## EXAMPLE 5

This example discloses the fabrication of a vascular prosthesis tube having a gradation of porosity radially across the wall.

A blend was formed between 65% by weight of a polyether based PU (Estane 58300 ex Goodrich) and 35% by weight of a polypropylene (Propathene PXC 31631). The polyurethane was dried under vacuum for 4 hours at 62°C. The polyurethane had a melt viscosity of 15620 poise at 190°C and the polypropylene had a melt viscosity of 2017 poise at 190°C. The blends were spun at a temperature of 190°C through a double C spinneret at a throughput of 15.8 g/min. The double C spinneret had dimensions of 2.33 mm internal radius, 0.21 mm slot width and a gap separation of 0.55 mm. The tube was quenched using a conventional air quench.

500 mg samples were immersed in 80 cc of boiling petroleum for 90 minutes.

Substantially all of the polypropylene was extracted from the wall of the tube. In cross section the inner edge comprised of spaced fibrils of polyurethane which were substantially aligned to the axis of the tube, such aligned fibrils being interconnected to each other in a random manner. The outer edge formed a structure with low porosity. Thus the degree of porosity varies across the wall, decreasing radially outwards. This asymmetry imparts strength to the tube while still maintaining its high elasticity and porous nature. The interior structure of the wall is shown in the accompanying photomicrograph Fig 5.

It will be realised that through the polyurethanes used in the above Examples are members of the class of polyurethanes which we have said are particularly desirable, it should be understood that they are not necessarily biocompatible and in practice it would be necessary to use such a biocompatible polymer.

## Claims

1. A porous tubular, substantially cylindrical synthetic vascular prosthesis, the wall of which has a structure comprising spaced fibrils of a synthetic polymeric material characterised in that (1) the tube has been produced by melt spinning the polymeric material and (2) the spaced fibrils of synthetic polymeric material are substantially aligned with the axis of the tube, such aligned spaced fibrils being interconnected to each other in a random manner.

2. A melt spun vascular prosthesis as claimed in claim 1 of a polyether based polyurethane.

3. A melt spun vascular prosthesis as claimed in claim 2 in which the polyether based polyurethane is one which is based on poly-(tetrahydrofuran), methylene bis (4-phenylisocyanate) and 1,4-butane diol.

4. A melt spun vascular prosthesis as claimed in claim 1 in which the synthetic polymeric material is biodegradable.

5. A melt spun vascular prosthesis as claimed in claim 1 in which the tube has a gradation of porosity across its wall in a radial direction.

## Revendications

1. Prothèse vasculaire synthétique tubulaire sensiblement cylindrique poreuse dont la paroi a une structure qui comprend des fibrilles espacées d'une matière polymère synthétique, caractérisée en ce que (1) le tube a été produit par filage à l'état fondu de la matière polymère et (2) les fibrilles espacées de matière polymère sont sensiblement alignées suivant l'axe du tube, ces fibrilles espacées et alignées étant connectées les unes aux autres au hasard.

2. Prothèse vasculaire filée à l'état fondu suivant la revendication 1, faite d'un polyuréthanne à base de polyéther.

3. Prothèse vasculaire filée à l'état fondu suivant la revendication 2, dans laquelle le polyuréthanne à base de polyéther est un polyuréthanne à base de polytétrahydrofuranne, de méthylène bis (4-phénylisocyanate) et de 1,4-butanediol.

4. Prothèse vasculaire filée à l'état fondu suivant la revendication 1, dans laquelle la matière polymère synthétique est biodégradable.

5. Prothèse vasculaire filée à l'état fondu suivant la revendication 1, dans laquelle le tube présente une gradation de porosité en direction radiale dans sa paroi.

## Ansprüche

1. Poröse, rohrförmige, im wesentlichen zylindrische synthetische Gefäßprothese, deren Wandung eine Struktur besitzt, die im Abstand angeordnete Fibrillen aus einem synthtischen polymeren Material aufweist, dadurch gekennzeichnet, daß (1) das Rohr durch Schmelzspinnen des polymeren Materials hergestellt worden ist und (2) die im Abstand angeordneten Fibrillen aus synthetischem polymeren Material im wesentlichen mit der Achse des Rohrs ausgerichtet sind, wobei die ausgerichteten, im Abstand angeordneten Fibrillen in einer statistischen Weise miteinander verbunden sind.

2. Schmelzgesponnene Gefäßprothese nach Anspruch 1 aus einem Polyether, der auf einem Polyurethan basiert.

3. Schmelzgesponnene Gefäßprothese nach Anspruch 2, bei welcher der auf einem Polyurethan basierende Polyether ein solcher ist, der auf Poly-(tetrahydrofuran), Methylen- bis(4-phenylisocyanat) und 1,4-Butandiol basiert.

4. Schmelzgesponnene Gefäßprothese nach Anspruch 1, bei welcher das synthetische polymere Material biologisch abbaubar ist.

5. Schmelzgesponnene Gefäßprothese nach Anspruch 1, bei welcher das Rohr durch seine Wandung in radialer Richtung eine Graduierung der Porosität aufweist.

*Fig.1.*

*Fig.5.*

## Fig.2A.

## Fig.2B.

Fig. 3.

Fig. 4.